# NEW EUROPEAN PATENT SPECIFICATION

(11) **EP 2 600 916 B2**
(45) Date of publication and mention of the opposition decision: **11.04.2018**
(45) Mention of the grant of the patent: 14.01.2015
(21) Application number: 11745663.2
(22) Date of filing: 05.08.2011
(51) Int. Cl.: A61M 1/00

(54) **SYSTEM FOR TREATING TISSUE OF A PATIENT USING A THERMOELECTRIC GENERATOR**
SYSTEM ZUR GEWEBEBEHANDLUNG BEI EINEM PATIENTEN MIT EINEM THERMOELEKTRISCHEN GENERATOR
SYSTÈME DE TRAITEMENT DES TISSUS CHEZ UN PATIENT UTILISANT UN GÉNÉRATEUR THERMOÉLECTRIQUE

(30) Priority: 06.08.2010 US 371496 P
(43) Date of publication of application: 12.06.2013
(73) Proprietor: KCI Licensing, Inc., San Antonio, TX 78265 (US)
(72) Inventor: LOCKE, Christopher, Brian, Bournemouth BH9 3SD (GB); COULTHARD, Richard, Daniel John, Verwood BH34 6LL (GB)
(74) Representative: CMS Cameron McKenna Nabarro Olswang LLP
(86) International application number: PCT/US2011/046832
(87) International publication number: WO 2012/019147

(56) References cited:
- WO-A1-2009/004370
- GB-A- 2 442 132
- US-A- 5 149 331
- US-A- 6 096 966
- US-A1- 2002 120 185
- US-A1- 2005 049 578
- US-A1- 2008 065 172
- US-A1- 2009 082 835
- US-A1- 2009 171 404
- US-A1- 2009 196 855
- US-A1- 2010 042 074
- US-B1- 6 304 521
- US-B2- 7 160 316
- US-B2- 7 449 614
- US-B2- 7 532 937
- US-B2- 7 687 704
- Thermoelectric effet-Wikipedia (31.07.2010)
- Thermoelectric Generators on Living Beings, Leonov et al (September 2007)

## Description

### BACKGROUND

Technology of tissue treatment systems that provide reduced pressure for treating tissue sites, such as wounds, of patients has been significantly improved in recent years. Pump, dressing, and drape designs have all improved in quality, size reduction, and/or efficiency. While the above aspects of tissue treatment systems and components have improved, power source technology for powering pumps of the tissue treatment systems have only marginally improved. Even as newer pump designs have improved in reducing power draw, ultimately, a limiting factor of portable tissue treatment systems and non-portable tissue treatment systems is power supply duration and power consumption in general.

US2002/120185 discloses a method and apparatus for the transcutaneous monitoring of blood gases generally comprises a blood gas data acquisition device, a vacuum source and a blood gas transducer unit. The blood gas transducer unit is adapted for application to a patient's skin and administration of a local vacuum at the area of patient application.

### SUMMARY

### BRIEF DESCRIPTION

There is provided a system for treating a tissue site of a patient using reduced pressure in accordance with claim 1. A selection of optional features are set out in the dependent claims.

Illustrative embodiments of the present invention are described in detail below with reference to the attached drawing figures, which are incorporated by reference herein and wherein:
FIG. 1 is an illustration of an illustrative electronic circuit model of a traditional mechanical thermoelectric generator;
FIG. 2 is a schematic of an illustrative semiconductor thermoelectric generator;
FIG. 3 is an illustration of an illustrative tissue treatment system including a drape for use in covering a tissue site of a patient and thermoelectric generators for use in charging a power source of a tissue treatment system;
FIG. 4 is an illustration of another illustrative tissue treatment system and drape for treating a tissue site of a patient;
FIG. 5A is an illustration of a patient wearing an illustrative tissue treatment system combined with a band for use in treating a tissue site on an arm of the patient;
FIG. 5B is an illustration of an illustrative band configured with a tissue treatment system and thermoelectric generators integrated with the band;
FIG. 6 is an illustration of an illustrative thermoelectric generator for use in generating electricity to power a power source of a tissue treatment system configured to treat a tissue site of a patient;
FIG. 7 is an illustration of a thermoelectric generator configured to operate as a tubular conduit for passing exudate fluid of a patient therethrough and generating electricity in response to the temperature of the exudate fluid;
FIG. 8 is a flow diagram of an illustrative process for manufacturing a tissue treatment system with a thermoelectric generator; and
FIG. 9 is a flowchart of an illustrative process for treating a tissue site of a patient.

### DETAILED DESCRIPTION

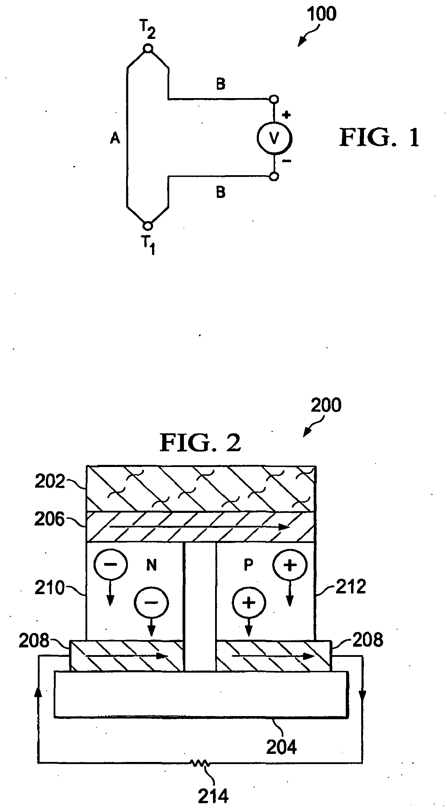

FIG. 1 is a schematic of an illustrative circuit diagram model 100 of a traditional mechanical thermoelectric generator. Thermoelectric generators generate electricity based on the principles of the Seebeck effect, which is a process that converts temperature differentials directly into electricity. A temperature differential sensed by a thermoelectric generator produces a voltage or current. The temperature difference may be sensed by two different metals, or semiconductors in the case of a semiconductor design (see FIG. 2), which causes a continuous current to flow in the conductors if the conductors form a complete loop. The Seebeck effect has been used for many years to form thermocouples. Several thermocouples, when connected in series, are called a thermopile, which is constructed in order to increase the output voltage since the voltage induced over each individual thermocouple is small. The electrical model 100 is shown to include two temperature levels, T₁ and T₂. Metals A and B may be used to form an electrical loop to create a voltage, as shown. The voltage may be computed by using the Seebeck coefficients and temperature differential, as provided by the following equation: V=(SB-SA)(T₂-T₁), where SA and SB are the Seebeck coefficients or thermoelectric power of the metals A and B as a function of temperature, and T₁ and T₂ are the temperatures of the two junctions between the metals A and B.

With regard to FIG. 2, a schematic of an illustrative thermoelectric generator 200 represents a semiconductor thermoelectric generator that provides a more efficient and effective way to generate electricity from sensing temperature differentials than using different metals, as described in FIG. 1. As shown, a heat source 202, such as a patient's skin or tissue, may be used as a first temperature level and a cool side 204 that senses a second temperature level, such as a temperature level provided by ambient temperature, ice pack, or other temperature or coolant source, may provide a temperature differential with respect to the heat source 202 and be used to generate electricity by the thermoelectric generator 200. Between the heat source 202 and cool side 204, thermoconductive materials 206 and 208 may sandwich semiconductor material 210 and 212, which may include an n-type semiconductor material and p-type semiconductor material, respectively. In operation, the heat source 202 drives electrons in the n-type element toward the cool side 204, thus creating a current through the circuit. Holes in the p-type element flow in the direction of the current, thereby enabling the current to be used to power a load 214, and, thus, converting the thermal energy into electrical energy. In one embodiment, the load 214 may be a power source or part of a power source (e.g., electronic circuit) that is used to charge up the power source. In one embodiment, the power source may be a rechargeable battery and, optionally, include an electronic circuit used to receive electricity and recharge the rechargeable battery, as understood in the art. It should be understood that the configuration of the thermoelectric generator 200 is illustrative, and alternative configurations, such as changing the positions of the heat source 202 and cool side 204.

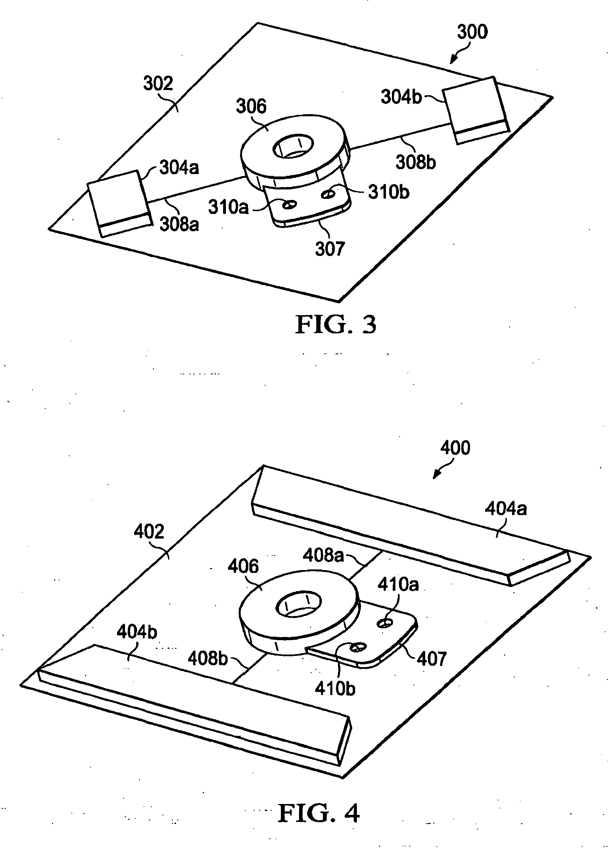

With regard to FIG. 3, an illustration of an illustrative tissue treatment system 300 is shown to include a drape 302 that is configured to overlay a tissue site of a patient that is being treated by the tissue treatment system 300. In one embodiment, the tissue treatment system may include one or more thermoelectric generators 304a-304b (collectively 304) that are used to generate electricity by sensing a temperature differential between a temperature level of tissue of a patient, for example, and another temperature level, such as ambient temperature of a room. The thermoelectric generators 304 are shown to include surfaces that are placed in contact with skin of a patient so as to operate at heat sources, while surfaces opposite the surfaces placed in contact with the skin may be used as cold sides so as to create a temperature differential to cause the thermoelectric generators 304 to generate electricity.

The tissue treatment system 300 may also include a pump unit 306, which, as shown, is a micro-pump or disc pump, as understood in the art, which operates to provide a reduced pressure at the tissue site. The reduced pressure at the tissue site, as understood in the art, helps generate tissue growth to improve or stimulate tissue healing. A control unit 307 may be configured with a processing unit or other control electronics (not shown) that controls and drives the pump unit 306 to generate the reduced pressure at the tissue site. The thermoelectric generators 304 may be electrically coupled to conductors 308a-308b (collectively 308) that conduct electricity generated by the thermoelectric generators 304 to the control unit 307. The control unit 307 may include one or more power sources 310a-310b (collectively 310) that may be used to drive power to the control unit 307 and pump unit 306. In one embodiment, the control unit 307, as part or separate from the power sources 310, may include circuitry (not shown) that, at least in part, utilizes electricity collected by the thermoelectric generators 304 by combining the collected electricity with the power sources 310 (e.g., by using an electronic summer) or to charge the power sources 310 while in operation and/or while not being used. In one embodiment, the power sources 310 are rechargeable batteries. In anther embodiment, the power sources 310 are capacitive elements. It should be understood that the principles of the present invention may be applied to any tissue treatment system that utilizes an electrically powered device, such as the pump unit 306.

As shown, the thermoelectric generators 304, pump unit 306, and control unit 307 may be configured to be small enough to be positioned within the confines of the drape 302. Alternatively, the configuration of the thermoelectric generators 304, pump unit 306, and control unit 307 may be positioned above the drape. In such an out-of-drape configuration, thermal conductors, such as surfaces of metal conductors, may extend through the drape so as to contact skin of the patient or, alternatively, reside on top of the drape and collect whatever heat passes through the drape for the heat source side of the thermoelectric generator. Other configurations in which the thermoelectric generators may be positioned at (i.e., under, above, integrated with, or near) the drape 302 may be utilized in accordance with the principles of the present invention. It should be understood that thermal conductors in a wide variety of configurations, rigid or flexible, may be utilized in accordance with the principles of the present invention. In one embodiment, the drape may be configured to allow the pump unit 306 to have at least a portion extend through the drape 302 with an airtight seal with the pump unit 306 so that fluid, such as air, may be discharged from beneath the drape 302 when sealed at a tissue site of a patient. In another embodiment in which the pump unit 306 is positioned on top of the drape 302, an inlet valve opening may be aligned with an opening (not shown) through the drape 302 to draw fluid from a pocket formed by the drape 302 and the patient. To reduce or eliminate the possibility of exudate fluid from entering the pump unit 306, a hydrophobic filter may be positioned in front of an inlet valve to prevent exudate from entering the pump unit 306. Additional description may be found in co-pending U.S. Patent Application Serial No. 12/824,604 filed June 28, 2010, which is incorporated herein by reference in its entirety.

A variety of drape configurations are contemplated. As shown, the drape may be configured with conductors 308 that extend along a surface of the drape 302. The conductors 308 may be positioned on top, bottom, or through the drape 302. In one embodiment, the conductors are printed on the drape 302. The thermoelectric generators 304 may be fixedly attached or removably attached to the drape 302. If fixedly attached, the thermoelectric generators 304 may be disposable so that upon completed use of the drape 302, the drape 302 and thermoelectric generators 304 are disposed with other biomedical waste. If removably attached, the thermoelectric generators 304 are formed in a manner so as to be washable and capable of being sterilized for re-use with the same or other patients. In the thermoelectric generators 304 are removable, then the drape 302 may be configured with connectors (not shown) onto which the thermoelectric generators 304 may be attached to provide power via the conductors 308 to the pump unit 306. The connectors may be snaps, clips, or other conductive connector onto which the thermoelectric generators 304 may be attached for securing to the drape 302 and deliver electricity that has been generated. The pump unit 306 may also be fixedly or temporarily attached to the drape 302 in the same or similar manner as the thermoelectric generators 304.

With regard to FIG. 4, an illustrative tissue treatment system 400 may include a drape 402 that has thermoelectric generator film sheets 404a and 404b (collectively 404) that may be attached or applied to the drape 402. Although shown as two film sheets 404, other numbers of film sheets may be utilized. The film sheets 404 may be single or multiple layers of sheets, and may be metallic or other material capable of thermal conduction. The film sheets 404 may have additional materials and/or electrical components that are capable of generating electricity, as described with regard to FIGS. 1 and 2. The film sheets 404 may be adhered or otherwise attached to the skin-side surface of the drape 402. The tissue treatment system 400 may also include a pump unit 406 and control unit 407 that are used to generate reduced pressure at a tissue site for promoting tissue growth of the tissue site, as understood in the art. Conductor lines 408a and 408b (collectively 408) may provide for electricity to be carried between the thermoelectric generator film sheets 404a and 404b to the control unit 407 for use in providing electricity or power to power sources 410a and 410b, as described with regard to FIG. 3. It should be understood that the power sources 410a and 410b may be a single power source or multiple power sources that are recharged using the same or different circuitry and that the conductor lines 408 may be configured to cause the power sources to operate in series.

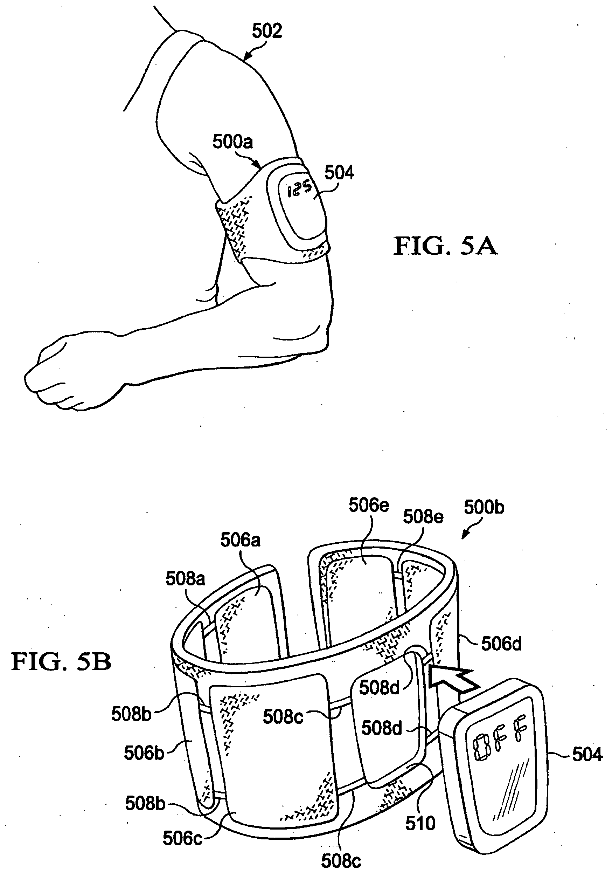

With regard to FIG. 5A, an illustration of a band 500a is shown to be wrapped around an arm of a patient 502. The band 500a is further shown to be configured with a tissue treatment system 504 that is used to generate reduced pressure for treating a tissue site of the patient 502. The band 500a may be configured with thermoelectric generators (not shown) that sense temperature of the patient's arm at or near the tissue site to generate electricity in response to a differential temperature of the tissue of the patient's arm and a second temperature level, which may be ambient temperature or any other temperature level (e.g., ice pack in contact with the thermoelectric generators). Being near or local to a tissue site may include being on a body part on which a tissue site exists (e.g., arm, leg, abdomen, etc.). It should be understood that the band 500a may be configured to be wrapped around any body part. It should further be understood that the band 500a may alternatively be configured more in the fashion of a bandage with adhesive for sticking to a patient as opposed to wrapping around a patient, thereby enabling an easier installation for a patient's torso.

With regard to FIG. 5B, an illustration of an illustrative band 500b is shown to include a tissue treatment system 504 and thermoelectric generators 506a-506d (collectively 506) and conductors 508a-508e (collectively 508) attached to the band 500b. The conductors 508 are utilized for conducting electricity generated by the thermoelectric generators 506 in series or in parallel to the tissue treatment system 504. The tissue treatment system 504 may include a power source (not shown) that is used to power the tissue treatment system 504 and, in response to receiving electricity from the thermoelectric generators 506 via the conductors 508, charge the power source. An opening 510 in the band 500b may be configured to receive the tissue treatment system 504. In the same or analogous manner, the band 500b may include openings (not shown) into which the thermoelectric generators are positioned that allow one side of the thermoelectric generators to directly contact tissue (e.g., skin) or indirectly contact the tissue (e.g., via foam, gauze, mesh, or any other material) of a patient and another side to be exposed to ambient temperature or other differential temperature source (e.g., ice pack). Alternatively, the thermoelectric generators may be positioned within pockets in the band 500b that allow for differential temperature sensing to cause electricity to be generated. The tissue treatment system 504 may alternatively be fixedly attached to the band 500b, and the thermoelectric generators 506 may be fixedly attached to the band 500b, as well. Alternatively, the thermoelectric generators 506 and tissue treatment system 504 may be temporarily attached to the band 500b via electrically conductive connectors, such as snaps (not shown), which may be in electrical communication with the conductors 508 for providing electricity flow between the thermoelectric generators 506 and tissue treatment system 504 for use in powering a pump by charging a battery or otherwise, as described herein. Other mechanical fastening mechanisms may be utilized to secure the thermoelectric generators 506 to the band 500b.

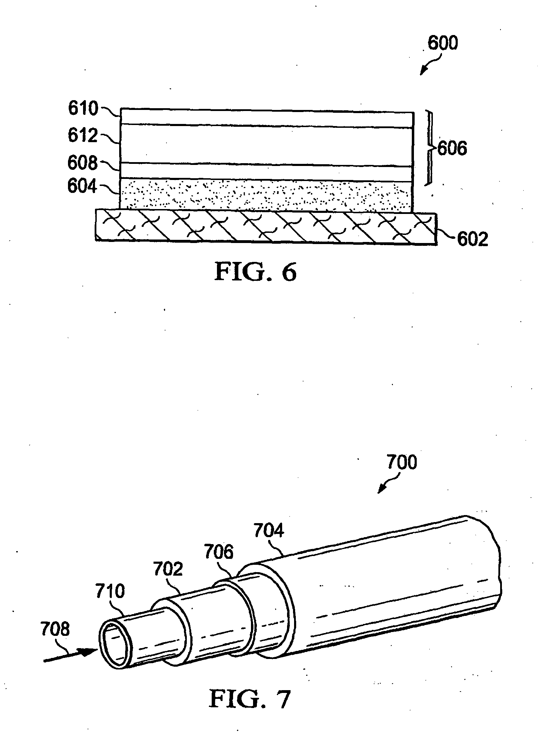

With regard to FIG. 6, an illustration of an illustrative tissue site 600 is shown to include tissue 602 of a patient having a wound dressing member 604, such as a foam dressing, disposed thereon with a thermoelectric generator 606 being disposed on top of the wound dressing member 604. In this embodiment, the wound dressing member 604 operates to collect exudate from the tissue 602, thereby conducting heat to the theremoelectric generator 606. If a drape is included, then the drape may be disposed above or below the thermoelectric generator 606. If the drape is positioned below the thermoelectric generator 606, then a vent that provides for an air tight seal with the thermoelectric generator 606 may provide for reduced pressure at the tissue 602 to be accomplished by use of a vacuum pump, for example, and direct thermal contact to be made between the wound dressing member 604 and the thermoelectric generator 606. Although the thermoelectric generator 606 is not directly in contact with the tissue 600 of the patient, the foam dressing 604 and exudate fluid that is absorbed into the wound dressing member 604 is capable of operating as a heat transfer element to transfer heat produced by the patient to a "hot" side 608 of the thermoelectric generator 606. The thermoelectric generator 606 may also include a "cold" side 610 that senses ambient temperature of a room or other temperature source. A semiconductor material 612 may provide for electricity generation by sensing the temperature differential between the hot side 608 and cold side 610 of the thermoelectric generator 606, as described with regard to FIG. 2. By having the thermoelectric generator 606 disposed on top of the foam 604, the tissue treatment system may be more compact than if disposed near the tissue site, since being near the tissue site would cause the thermoelectric generator 606, which is part of the tissue treatment system, to have more horizontal area than if disposed on top of the foam 604. Being near the tissue site means to be disposed at surrounding tissue from a tissue site (e.g., wound) that is being treated with a drape covering.

In an alternative embodiment, the exudate fluid that is collected may be applied to one or more chemicals, such as iron or copper salts, that produce an exothermic reaction to provide a heat source for the thermoelectric generator 606. The application of the exudate fluid may simply include adding the exudate fluid to a canister with the chemical(s). In one embodiment, the exudate fluid may be mechanically mixed with the chemical(s). In one embodiment, the exothermic reaction may be performed in a canister (not shown) or in-line, as shown in FIG. 6. Being in-line means that the thermoelectric generator is positioned in a flow path of exudate fluid, which may include being positioned above a tissue site, as shown in FIG. 6. In one embodiment, the thermoelectric generator may be formed in a particular shape that forms the in-line flow path or conduit, such as being configured in a tubular form or any other shape, through which the exudate fluid flows so that the exudate fluid has an opportunity to interact with the thermoelectric generator. In the case of the thermoelectric generator being in a tubular form or configured to fit within a tube, the thermoelectric generator may be positioned as an inside member so that an outside member may operate to contact a coolant source, thereby allowing for a temperature differential. In the case of the thermoelectric generator using a reagent, such as a chemical, various structures, such as being disposed within a foam, mesh, or other porous material, may be utilized to allow the exudate to contact the reagent along the exudate flow path. Being in a canister may include being fixedly or non-fixedly positioned in a collection canister for the exudate fluid. It should be understood that a variety of different canister and in-line configurations for integrating the exudate fluid with the chemicals may be utilized to produce heat that is used to generate electricity utilizing a thermoelectric generator in accordance with the principles of the present invention.

With regard to FIG. 7, an illustration of a thermoelectric generator 700 configured in the shape of a conduit, in this case a tubular conduit, is shown. The thermoelectric generator 700 may have a hot core 702 surrounded by a cold face 704. Between the hot core 702 and cold face 704, semiconductor material 706 that is appropriately doped, as described with regard to FIG. 2, may be utilized to generate electricity as a result of exudate fluid 708 or wound fluid that is hotter than the ambient temperature or other temperature being sensed by the cold face 704. In one embodiment, a disposable fuel core 710 may be used to protect the hot core 702 from becoming contaminated by patients. The disposable fuel core 710 may have a thermal coefficient that allows the temperature level of the exudate fluid 708 to be sensed by the hot core 702, thereby providing a temperature differential and causing the thermoelectric generator to generate electricity for use by the tissue treatment system in generating reduced pressure to a tissue site. Although not shown, the thermoelectric generator 700 may be electrically attached to a tissue treatment system that uses a pump that delivers reduced pressure at a tissue site via conduit(s) and removes fluid from the tissue site. Electrical energy produced by the thermoelectric generator 700 may reduce power consumption of the tissue treatment system.

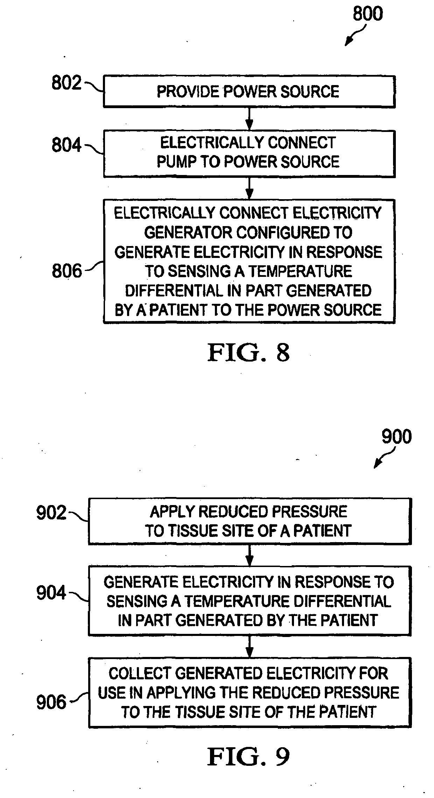

With regard to FIG. 8, a flow chart of an illustrative process 800 for manufacturing a tissue treatment system is shown. The process 800 starts at step 802, where a power source is provided. The power source may include a rechargeable battery, energy storage unit, such as a capacitor, or any other power source, including an AC-to-DC converter to convert power being delivered from a wall socket, as understood in the art. At step 804, a pump may be electrically connected to the power source. It should be understood that rather than using a pump, any electrically powered device used to treat tissue of a patient may be utilized. At step 806, an electricity generator configured to generate electricity in response to sensing a temperature differential in part generated by a patient may be electrically connected to the power source. By electrically connecting the electricity generator to the power source, electricity generated by the electricity generator may be used to provide additional power to the power source during active operation or charge the power source either while the tissue treatment system is operating or charge the power source while not in operation. Although described herein as generating electricity for use in applying a reduced pressure, it should be understood that the generated electricity be used to power components other than a pump, such as a clock, computing unit, electronic display, and/or other electronic components(s), and still be considered to be used in applying a reduced pressure since additional power will be available for the pump that would otherwise be used for powering the other component(s).

With regard to FIG. 9, a flow chart of an illustrative process 900 of a tissue treatment system for treating a tissue site of a patient is shown. At step 902, reduced pressure may be applied to the tissue site of a patient. In applying the reduced pressure to the tissue site, a pump may be utilized to reduce pressure at the tissue site that is covered by a drape, as understood in the art. At step 904, electricity may be generated in response to sensing a temperature differential in part generated by the patient. In one embodiment, the patient's skin or other tissue that provides heat may be sensed by a thermoelectric generator to form a temperature differential between the temperature level of the patient's tissue and other temperature or coolant source, such as ambient room temperature, ice pack, or other coolant source. At step 906, the generated electricity may be collected for use in applying the reduced pressure to the tissue site of the patient. The collected electricity may be collected in a power source, such as a rechargeable battery or capacitor, and be used to power a pump, such as a micro-pump, that is being used to reduce pressure at the tissue site of the patient.

The previous detailed description is of a small number of embodiments for implementing the invention and is not intended to be limiting in scope. One of skill in this art will immediately envisage the methods and variations used to implement this invention in other areas than those described in detail. The following claims set forth a number of the embodiments of the invention disclosed with greater particularity.

## Claims

1. A system (300, 400) for treating a tissue site of a patient using reduced pressure, said system comprising:
a pump (306, 406) configured to generate reduced pressure at the tissue site; and **characterised by**
a thermoelectric generator (200, 304, 404, 606, 700) configured to generate electricity in response to the thermoelectric generator (200, 304, 404, 606, 700) sensing a temperature differential of a first temperature level and a second temperature level, the first temperature level being generated by the patient, the generated electricity being supplied to a system (300, 400) utilized to supply the reduced pressure,
further comprising an electrically powered device for supplying reduced pressure to the tissue site, the electrically powered device including the pump (306, 406), wherein the electricity from the thermoelectric generator (200, 304, 404, 606, 700) is utilized to at least partially power the pump (306, 406).

2. The system according to claims 1, wherein a first side of the thermoelectric generator (200, 304, 404, 606, 700) is configured to be in contact with tissue of the patient.

3. The system according to claim 1 or claim 2, wherein the second temperature level is provided by a coolant source.

4. The system according to claim 1 or claim 2, wherein the second temperature is provided by exudate from the tissue site.

5. The system according to claim 4, wherein the exudate is reacted with a reagent such that an exothermic reaction occurs.

6. The system according to any of claims 1 to 5, further comprising a drape for creating a sealed space at the tissue site, wherein the thermoelectric generator (200, 304, 404, 606, 700) is at least partially located on the drape.

7. The system according to claim 6, wherein the thermoelectric generator (200, 304, 404, 606, 700) includes at least one thermoelectric generator sheet positioned on the drape.

8. The system according to claim 1 or claim 2, further comprising a dressing member, wherein the thermoelectric generator (200, 304, 404, 606, 700) is located on the dressing member.

9. The system according to any of claim 1 to 5, wherein the thermoelectric generator (700) is configured to be a tubular shape to provide a conduit for exudate from the tissue site, the inner surface of the conduit sensing the first temperature level, and an outer surface of the conduit sensing the second temperature level.

10. The system according to claim 9, wherein the thermoelectric generator (700) further comprising a disposable core to protect the thermoelectric generator from the exudate.

11. The system according to any of claim 1 to 10, wherein the thermoelectric generator (200, 304, 404, 606, 700) is a semiconductor thermoelectric generator.

## Patentansprüche

1. System (300, 400) zur Behandlung einer Gewebestelle eines Patienten unter Verwendung von Unterdruck, wobei das System umfasst:
eine Pumpe (306, 406), die so ausgestaltet ist, dass sie an der Gewebestelle Unterdruck erzeugt; und **gekennzeichnet ist durch**
einen thermoelektrischen Generator (200, 304, 404, 606, 700), der so ausgestaltet ist, dass er als Reaktion auf die Erfassung einer Temperaturdifferenz zwischen einem ersten Temperaturniveau und einem zweiten Temperaturniveau durch den thermoelektrischen Generator (200, 304, 404, 606, 700) Elektrizität erzeugt, wobei das erste Temperaturniveau durch den Patienten erzeugt wird, wobei die erzeugte Elektrizität einem zur Zufuhr des Unterdrucks eingesetzten System (300, 400) zugeführt wird,
weiterhin umfassend eine elektrisch angetriebene Vorrichtung zur Zufuhr von Unterdruck an die Gewebestelle, wobei die elektrisch angetriebene Vorrichtung die Pumpe (306, 406) umfasst, wobei die Elektrizität von dem thermoelektrischen Generator (200, 304, 404, 606, 700) zum wenigstens teilweisen Antrieb der Pumpe (306, 406) eingesetzt wird.

2. System nach Anspruch 1, wobei eine erste Seite des thermoelektrischen Generators (200,304,404, 606, 700) so ausgestaltet ist, dass sie mit Gewebe des Patienten in Kontakt ist.

3. System nach Anspruch 1 oder Anspruch 2, wobei das zweite Temperaturniveau durch eine Kühlmittelquelle bereitgestellt wird.

4. System nach Anspruch 1 oder Anspruch 2, wobei die zweite Temperatur durch Exsudat von der Gewebestelle bereitgestellt wird.

5. System nach Anspruch 4, wobei das Exsudat derart mit einem Reagens zur Reaktion gebracht wird, dass eine exotherme Reaktion erfolgt.

6. System nach einem der Ansprüche 1 bis 5, weiterhin umfassend eine Abdeckung zur Schaffung eines abgedichteten Raums an der Gewebestelle, wobei sich der thermoelektrische Generator (200, 304, 404, 606, 700) wenigstens teilweise auf der Abdeckung befindet.

7. System nach Anspruch 6, wobei der thermoelektrische Generator (200, 304, 404, 606, 700) wenigstens ein auf der Abdeckung angeordnetes, dem thermoelektrischen Generator zugehöriges Plättchen umfasst.

8. System nach Anspruch 1 oder Anspruch 2, weiterhin umfassend ein Verbandelement, wobei sich der thermoelektrische Generator (200, 304, 404, 606, 700) auf dem Verbandelement befindet.

9. System nach einem der Ansprüche 1 bis 5, wobei der thermoelektrische Generator (700) rohrförmig ausgestaltet ist, so dass er eine Leitung für Exsudat von der Gewebestelle bereitstellt, wobei die Innenfläche der Leitung das erste Temperaturniveau erfasst und eine Außenfläche der Leitung das zweite Temperaturniveau erfasst.

10. System nach Anspruch 9, wobei der thermoelektrische Generator (700) weiterhin einen zur Einmalverwendung bestimmten Kern zum Schutz des thermoelektrischen Generators vor dem Exsudat umfasst.

11. System nach einem der Ansprüche 1 bis 10, wobei der thermoelektrische Generator (200, 304, 404, 606, 700) ein halbleiterbasierter thermoelektrischer Generator ist.

## Revendications

1. Système (300, 400) pour traiter un site tissulaire d'un patient sous pression réduite, ledit système comprenant :
une pompe (306, 406) configurée pour générer une pression réduite sur le site tissulaire ; et
**caractérisé par** :
un générateur thermoélectrique (200, 304, 404, 606, 700) configuré pour générer de l'électricité en réponse au générateur thermoélectrique (200, 304, 404, 606, 700) qui détecte une différence de température d'un premier niveau de température et d'un second niveau de température, le premier niveau de température étant généré par le patient, l'électricité générée étant fournie à un système (300, 400) utilisé pour appliquer la pression réduite, comprenant en outre un dispositif électrique pour appliquer une pression réduite au site tissulaire, le dispositif électrique comprenant la pompe (306, 406), dans lequel l'électricité provenant du générateur thermoélectrique (200, 304, 404, 606, 700) est utilisée pour alimenter au moins en partie la pompe (306, 406).

2. Système selon la revendication 1, dans lequel un premier côté du générateur thermoélectrique (200, 304, 404, 606, 700) est configuré pour être en contact avec le tissu du patient.

3. Système selon la revendication 1 ou la revendication 2, dans lequel le second niveau de température est fourni par une source de réfrigérant.

4. Système selon la revendication 1 ou la revendication 2, dans lequel la seconde température est fournie par un exsudat provenant du site tissulaire.

5. Système selon la revendication 4, dans lequel on fait réagir l'exsudat avec un réactif tel qu'une réaction exothermique se produise.

6. Système selon l'une quelconque des revendications 1 à 5, comprenant en outre un champ opératoire pour créer un espace scellé sur le site tissulaire, dans lequel le générateur thermoélectrique (200, 304, 404, 606, 700) est au moins en partie situé sur le champ opératoire.

7. Système selon la revendication 6, dans lequel le générateur thermoélectrique (200, 304, 404, 606, 700) comprend au moins une feuille de générateur thermoélectrique positionnée sur le champ opératoire.

8. Système selon la revendication 1 ou la revendication 2, comprenant en outre un élément de pansement, dans lequel le générateur thermoélectrique (200, 304, 404, 606, 700) est situé sur l'élément de pansement.

9. Système selon l'une quelconque des revendications 1 à 5, dans lequel le générateur thermoélectrique (700) est configuré pour avoir une forme tubulaire pour former un conduit destiné à l'exsudat provenant du site tissulaire, la surface interne du conduit détectant le premier niveau de température, et une surface externe du conduit détectant le second niveau de température.

10. Système selon la revendication 9, dans lequel le générateur thermoélectrique (700) comprend en outre un coeur jetable pour protéger le générateur thermoélectrique de l'exsudat.

11. Système selon l'une quelconque des revendications 1 à 10, dans lequel le générateur électrique (200, 304, 404, 606, 700) est un générateur thermoélectrique à semi-conducteur.
